# EUROPÄISCHE PATENTANMELDUNG

(11) **EP 0 638 323 A1**
(43) Veröffentlichungstag der Anmeldung: **15.02.1995**
(21) Anmeldenummer: 94110946.4
(22) Anmeldetag: 14.07.1994
(51) Int. Cl.: A61M 5/32

(54) **Originalitätsverschluss für ein medizinisches Gerät**

(30) Priorität: 13.08.1993 DE 4327171
(71) Anmelder: B. BRAUN MELSUNGEN AG, D-34212 Melsungen (DE)
(72) Erfinder: Schnell, Joachim, Dr., D-34212 Melsungen (DE); Brethauer, Ulrich, D-34327 Körle (DE)
(74) Vertreter: Selting, Günther, Dipl.-Ing.

(57) **Zusammenfassung**

Das medizinische Gerät weist eine auf einem Ansatz (17;18) sitzende Schutzkappe (15;16) auf. Die Schutzkappe ist mit einem Siegel (20;22) auf dem Ansatz fixiert. Dieses Siegel besteht aus einer auf die angrenzenden Bereiche beider Teile fließfähig aufgebrachten und erhärteten Masse, vorzugsweise einem Schmelzkleber. Beim erstmaligen Abnehmen der Schutzkappe zerbricht das Siegel mit einem Knackgeräusch. Das Zerbrechen des Siegels ist optisch erkennbar. Das Siegel (20;22) ist einfach und schnell aufzubringen. Es erfordert keine konstruktiven Änderungen an dem medizinischen Gerät oder der Schutzkappe. Die Öffnungskraft zum Abnehmen der Schutzkappe wird nicht wesentlich erhöht.

## Beschreibung

Die Erfindung betrifft einen Originalitätsverschluß für ein medizinisches Gerät, das einen Ansatz aufweist, auf dem eine abnehmbare Schutzkappe sitzt.

Medizinische Geräte, die für den einmaligen Gebrauch bestimmt sind, wie z.B. Infusions- und Transfusionsgeräte sowie Kanülen sind üblicherweise an solchen Stellen, an denen Verletzungsgefahr besteht, oder an Verbindungsstellen, an denen der Anschluß an ein anderes Gerät erfolgt, mit einer Schutzkappe ausgerüstet, die auf einen Ansatz des Gerätes aufgesteckt ist. Die Schutzkappe schützt den Anwender vor Verletzungen und sie verhindert, daß die Umverpackung durch spitze Teile des Gerätes beschädigt wird. Eine weitere Wirkung der Schutzkappe besteht darin, daß sie die Innensterilität des Gerätes bewahrt, indem sie den Ansatz keimdicht umschließt. Ferner werden Beschädigungen der von der Schutzkappe umschlossenen Komponente verhindert. Derartige Schutzkappen sind üblicherweise auf das betreffende Bauteil reibschlüssig aufgesteckt, so daß sie sich einerseits unter Transportbedingungen nicht lösen, andererseits aber vom Anwender leicht abgenommen werden können. Diese Schutzkappen lassen sich beliebig oft abnehmen und wieder aufstecken.

Aus AT 394 950 B ist eine Injektionsspritze mit Nadelschutzkappe bekannt, bei der die Nadelschutzkappe aus einem mit der Injektionsspritze bleibend verbundenen Basisteil und einem abnehmbaren Kappenteil besteht. Zwischen Basisteil und Kappenteil befindet sich eine Sollbruchstelle. Bei dem erstmaligen Abnehmen des Kappenteils zerbricht die Sollbruchstelle, was nach außen hin sichtbar angezeigt wird. Eine derartige Originalitätssicherung ist aufwendig, weil sie erhebliche Änderungen an der Schutzkappe und einen zusätzlichen Materialeinsatz für das Basisteil erfordert. Außerdem muß das Basisteil an dem Kanülenansatz verschweißt oder verklebt werden.

EP 0 389 938 A2 beschreibt einen Originalitätsverschluß für eine Spritze, bei der auf dem Spritzenansatz eine abnehmbare Schutzkappe sitzt. Auf die Schutzkappe ist eine Überwurfhülse aufgeschoben, deren rückwärtiges Ende mit dem Spritzenkörper verklebt oder verschweißt ist. Durch Drehen der Überwurfhülse bricht die Klebung oder Schweißung, die jedoch von der Überwurfhülse verdeckt ist. Die Überwurfhülse hält sich dann nicht mehr auf der Schutzkappe. Das Fehlen der Überwurfhülse zeigt an, daß der Originalitätsverschluß bereits geöffnet wurde. Wenn es jedoch gelingt, die Überwurfhülse klemmend oder mit einem Klebtupfer erneut zu befestigen, kann die Tatsache, daß der Originaltitätsverschluß bereits aufgebrochen wurde, von außen nicht mehr erkannt werden.

Ein weiterer Originalitätsverschluß ist bekannt aus EP 0 440 047 A2. Hierbei ist an der Schutzkappe ein Stab angebracht, der über das Öffnungsende der Schutzkappe hinausragt und durch ein Loch eines von der Nadel des medizinischen Gerätes abstehenden Flansches hindurchgesteckt und hinter dem Loch zur Bildung einer Verdickung deformiert ist. Beim Abnehmen der Schutzkappe reißt der Stab, wodurch die Verdickung von der Rückseite des Flansches herabfällt und anzeigt, daß die Schutzkappe entfernt wurde. Ein reibschlüssiger Sitz der Schutzkappe auf dem Ansatz der Nadel ist hierbei nicht möglich.

Der Erfindung liegt die Aufgabe zugrunde, einen Originalitätsverschluß für ein medizinisches Gerät zu schaffen, der einfach und kostengünstig herstellbar ist.

Die Lösung dieser Aufgabe erfolgt erfindungsgemäß mit den im Anspruch 1 angegebenen Merkmalen.

Nach der Erfindung ist die Schutzkappe auf dem Ansatz mit einem Siegel fixiert, das auf die angegrenzenden Bereiche beider Teile fließfähig aufgebracht und anschließend erhärtet ist. Das Siegel besteht aus einer Siegelmasse, die vorzugsweise in Form eines Siegelpunktes aufgebracht ist. Als Siegelmasse eignen sich insbesondere Schmelzklebstoffe, die auch als Hot-Melt bezeichnet werden. Thermoplastische Schmelzklebstoffe sind im geschmolzenen Zustand gut benetzungsfähig. Sie entwickeln durch Wärmeabgabe ohne chemische Veränderung gute Festigkeiten zu thermoplastischen Kunststoffen, aus denen medizinische Geräte normalerweise hergestellt sind, aber auch zu elastomeren Polymerwerkstoffen.

Da medizinische Geräte in sehr hohen Stückzahlen produziert werden, ist es wichtig, daß der größte Teil der Festigkeit bereits nach Sekunden ohne Fixiereinrichtung und aufwendige Ofentrocknung erreicht wird, damit die fixierten Teile sofort weiterverarbeitet werden können. Im ausgehärteten Zustand wird das überlappend auf die beiden Teile aufgetragene Siegel zum Originalitätsverschluß. Beim erstmaligen Abnehmen der Schutzkappe, vorzugsweise durch eine Drehbewegung, vernimmt der Anwender ein deutliches Knackgeräusch, welches ihm Aufschluß über die bisherige Unversehrtheit des Siegels gibt. Auch optisch ist die Unversehrtheit des Siegels zu erkennen. Eine Verbesserung der optischen Erkennbarkeit kann dadurch erreicht werden, daß das Siegel aus einer eingefärbten Masse besteht. Wichtig ist, daß nur eine geringe Menge einer gut anhaftenden Siegelmasse zur Sicherung der Schutzkappe appliziert wird, damit der Anwender ohne größeren Kraftaufwand die Schutzkappe vom Gerät entfernen kann.

Der Originalitätsverschluß gibt dem Benutzer gleichzeitig die Gewähr, daß die Innensterilität des Gerätes gegeben ist. Die keimdicht auf dem Ansatz sitzende Schutzkappe verschließt einen Hohlraum, in dem sich beispielsweise eine Kanüle oder die Abdichtfläche eines Konnektors befindet. Das betreffende Teil wird von der Schutzkappe keimdicht umschlossen, solange das Gerät sich im Originalitätszustand befindet. Im Falle von Gammasterilisation erfolgt die Sterilisation durch die auf dem Gerät sitzende Schutzkappe hindurch. Bei Dampf- oder Gassterilisation ist vorzugsweise die Schutzkappe mit einem gasdurchlässigen Sterilfilter ausgestattet.

Im folgenden wird ein Ausführungsbeispiel der Erfindung unter Bezugnahme auf die Zeichnungen näher erläutert.

Es zeigen:

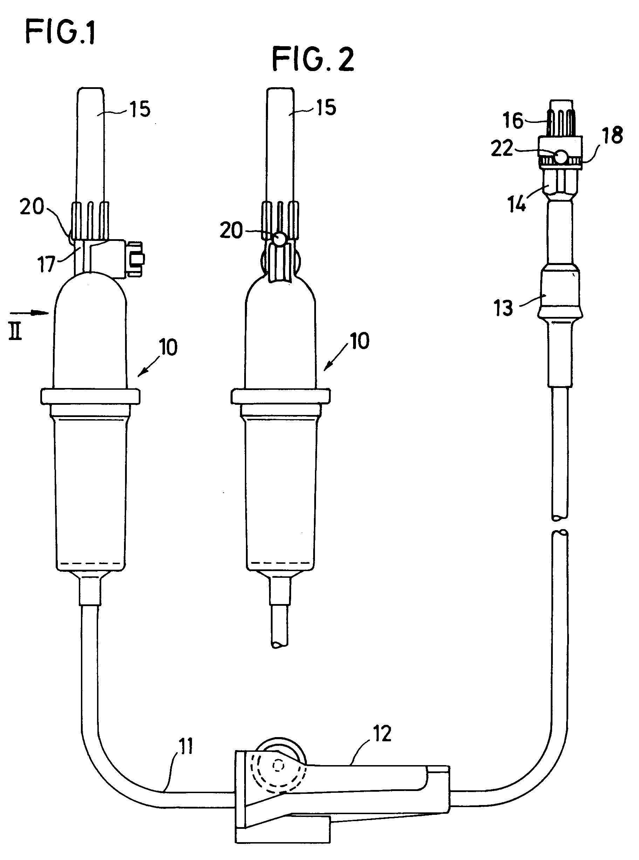

- Fig. 1: eine schematische Darstellung eines an seinen beiden Enden mit je einer Schutzkappe versehenen medizinischen Gerätes, und
- Fig. 2: eine Ansicht des Gerätes nach Fig. 1 aus Richtung des Pfeiles II.

Das dargestellte medizinische Gerät besteht aus einer Infusionsvorrichtung, die eine Tropfkammer 10, einen Schlauch 11 mit Rollenklemme 12, ein Injektionszwischenstück 13 und ein Verbindungsstück 14 aufweist. An den Konnektierungsstellen ist das medizinische Gerät jeweils mit einer Schutzkappe 15 bzw. 16 versehen. Die Schutzkappe 15 ist auf einen Ansatz 17 der Tropfkammer 10 reibend aufgesteckt und sie umschließt den (nicht dargetellten) Einstechdorn der Tropfkammer. Die Schutzkappe 16 ist auf einen Ansatz 18 des Verbindungsstücks 14 aufgesteckt, bei dem es sich um einen Luer-Ansatz handeln kann. Die Schutzkappe 16 schützt den Ansatz vor Kontaminationen. Sie kann abgenommen werden, um das Verbindungsstück 14 mit einem passenden Gegenstück zu verbinden.

Auf dem überlappenden Bereich von Schutzkappe 15 und Ansatz 17 ist ein punktförmiges Siegel 20 aus einem Schmelzklebstoff angebracht, das sich an einer einzigen Stelle des Umfangs befindet und einen Siegelpunkt bildet. In gleicher Weise ist ein Siegel 22 vorgesehen, das die Ränder der Schutzkappe 16 und des Ansatzes 18 überdeckt.

Für die Siegel 20 und 22 werden vorzugsweise Schmelzklebstoffe verwendet. Als besonders geeignet haben sich erwiesen die Schmelzklebstoffe
1. Lunatack P 120 K
   Polymerbasis: Ethylen/Vinylacetat (EVA)
   Hersteller: Fa. H.B. Fuller GmbH, München.
2. Ipatherm S14/216
   Polymerbasis: Polyurethan (PUR)
   Hersteller: Fa. H.B. Fuller GmbH, München.

Das Material 2. ist ein reaktiver PUR-Hot-Melt, der noch duroplastisch nachhärtet, was einer höheren Temperaturbeständigkeit des Brechsiegels zugute kommt. Prinzipiell kommen alle Schmelzklebstoffe auf Basis natürlicher oder synthetischer Polymere (Thermoplaste, Duroplaste und Elastomere) für das Siegel in Betracht. Es können aber auch Klebstoffe oder Lacke zum Einsatz kommen, die eine kurze Abbindezeit aufweisen, wie z.B. Sekundenkleber auf Cyanacrylatbasis, lichthärtende Epoxy-Kleber oder UV-härtende Kleber.

## Patentansprüche

1. Originalitätsverschluß für ein medizinisches Gerät, das einen Ansatz (17;14) aufweist, auf dem eine abnehmbare Schutzkappe (15;16) sitzt,
**dadurch gekennzeichnet,**
daß die Schutzkappe (15;16) auf dem Ansatz (17;18) mit einem Siegel (20;22) fixiert ist, das aus einer von außen auf die angrenzenden Bereiche beider Teile fließfähig aufgebrachten und erhärteten Masse besteht.

2. Originalitätsverschluß nach Anspruch 1, dadurch gekennzeichnet, daß das Siegel (20;22) aus einem Schmelzkleber besteht.

3. Originalitätsverschluß nach Anspruch 1 oder 2, dadurch gekennzeichnet, daß das Siegel (20;22) aus einem Siegelpunkt besteht, der nur an einer Stelle des Umfangs von Ansatz und Schutzkappe örtlich begrenzt vorgesehen ist.

4. Originalitätsverschluß nach einem der Ansprüche 1 bis 3, dadurch gekennzeichnet, daß das Siegel aus einer eingefärbten Masse besteht, deren Farbe sich von derjenigen des Ansatzes (17;18) und derjenigen der Schutzkappe (15;16) abhebt.
